(19) Europäisches Patentamt
European Patent Office
Office européen des brevets

(11) Veröffentlichungsnummer: **0 187 315 B1**

(12) **EUROPÄISCHE PATENTSCHRIFT**

(45) Veröffentlichungstag der Patentschrift: **17.04.91**

(51) Int. Cl.5: **A61K 31/495**, A61K 31/535, A61K 47/00, A61K 9/08

(21) Anmeldenummer: **85116001.0**

(22) Anmeldetag: **14.12.85**

Die Akte enthält technische Angaben, die nach dem Eingang der Anmeldung eingereicht wurden und die nicht in dieser Patentschrift enthalten sind.

(54) **Basische Zubereitungen von Chinoloncarbonsäuren.**

(30) Priorität: **05.01.85 DE 3500243**
**17.05.85 DE 3517709**

(43) Veröffentlichungstag der Anmeldung:
**16.07.86 Patentblatt 86/29**

(45) Bekanntmachung des Hinweises auf die Patenterteilung:
**17.04.91 Patentblatt 91/16**

(84) Benannte Vertragsstaaten:
**AT BE CH DE FR GB IT LI NL SE**

(56) Entgegenhaltungen:
**EP-A- 0 047 005**
**EP-A- 0 098 577**
**EP-A- 0 142 426**
**DE-A- 2 914 258**
**DE-A- 3 033 157**
**DE-A- 3 037 103**
**US-A- 4 352 803**
**US-A- 4 404 197**

**Gstirner, Einführung in die Verfahrenstechnik der Arzneiformung (1973), Seiten 276-277**

(73) Patentinhaber: **BAYER AG**

**W-5090 Leverkusen 1 Bayerwerk(DE)**

(72) Erfinder: **Naik, Arundev Haribhai**
**Walter-Flex-Strasse 20**
**W-5090 Leverkusen 1(DE)**
Erfinder: **Schlüter, Gerhard, Prof. Dr.**
**Gellertweg 35**
**W-5600 Wuppertal(DE)**
Erfinder: **Voege, Herbert, Dr.**
**Martin-Buber-Strasse 41**
**W-5090 Leverkusen(DE)**
Erfinder: **Grohe, Klaus, Dr.**
**Am Wasserturm 10**
**W-5068 Odenthal(DE)**

**EP 0 187 315 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft wäßrige basische Zubereitungen von Chinoloncarbonsäuren, gegebenenfalls in Form von Konzentraten, und ihre Verwendung zur Herstellung von Arzneimitteln, die zur Injektion, Infusion oder oralen Applikation geeignet sind.

Chinoloncarbonsäuren und ihre Analoga sind bekannt. Sie besitzen gute bakterizide Wirkung (vgl.EP-OS 67 666, DE-OS 2 914 258; DE-OS 3 142 854; 3 033 157; EP-OS 47 005; DE-OS 3 037 103).

Die Wirkstoffe sind jedoch nicht oder nur schwer wasserlöslich und lassen sich daher nicht oder nur schwer in Injektions- oder Infusionslösungen oder Lösungen zur oralen Applikation einsetzen. Da die Wirkstoffe sowohl basische als auch sauer reagierende Gruppen enthalten, ist bereits die Bildung von Salzen in den oben erwähnten Literaturstellen beschrieben. Salze mit Säuren sind zwar wasserlöslich. Doch neigen z.B. Hydrochloride beim Lagern zu Ausfällungen. Außerdem können Lösungen saurer Salze nicht mit elektrolytischen Lösungen wie z.B. phy siologischer Kochsalzlösung verdünnt werden, da es dabei ebenfalls zur Ausfällung der Wirkstoffe kommt.

Außerdem zeigen wäßrige Lösungen von Salzen der Chinoloncarbonsäuren mit Säuren beim Lagern Verfärbungen die auf eine geringe Photostabilität dieser Salze hinweisen.

Die Erfindung betrifft Infusions- und Injektionslösungen von Chinoloncarbonsäuren der allgemeinen Formel I oder II

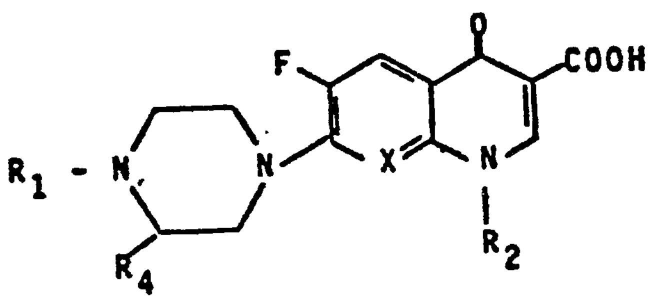
(I)

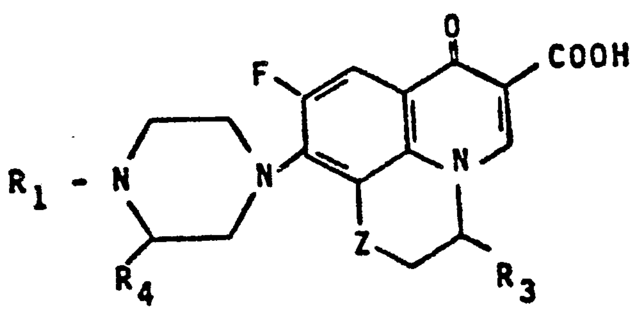
(II)

in denen

X N, C-H, C-F,
Z O oder $CH_2$,
$R_1$ Wasserstoff, Methyl, Ethyl oder $\beta$-Hydroxyethyl,
$R_2$ Cyclopropyl, 2-Fluorethyl oder Ethyl,
$R_3$ Wasserstoff oder Methyl und
$R_4$ H oder Methyl bedeutet,

die dadurch gekennzeichnet sind, daß sie die Verbindungen der Formel I oder II in Form ihrer Salze mit Basen in Gegenwart eines Überschusses an Base von 0,01 bis 100 ml/l, sowie gegebenenfalls in Gegenwart weiterer Hilfsstoffe enthalten und der pH der Zubereitungen zwischen 8 und 12,5 liegt.

Wäßrige basische Zubereitungen gemäß vorliegender Erfindung eignen sich als Injektions-, und Infusionslösungen.

Es war Überraschend, daß es bei den erfindungsgemäßen basischen Zubereitungen im Gegensatz zu den sauren Zubereitungen nicht zu Verfärbungen und Ausfällungen beim Lagern der Zubereitungen kommt.

Auch erweisen sich die erfindungsgemäßen basischen Zubereitungen als genauso geeignet zu Injektion

und Infusion wie die Zubereitungen die aus Salzen der Verbindungen der Formel I mit Säuren hergestellt worden sind. Dies konnte nach der Aussage in EP-OS 67 666 Seite 14 Zeile 16-17 nicht erwartet werden. In dieser Literaturstelle wird ausgeführt, daß die Natrium- und Cholin-Salze von Chinoloncarbonsäuren wegen ihres pH-Wertes nicht für parenterale Zubereitungen geeignet sind. Als Lösung des Problems werden dann Salze von Chinoloncarbonsäuren mit Galacturon-, Asparagin-, Glucon- oder Glutaminsäure angegeben. Die besondere Eignung basischer Salze für die Herstellung parenteraler Zubereitungen wurde nicht erkannt.

Bevorzugt werden Verbindungen der Formel I oder II verwendet in denen

X für N, C-H oder C-F steht,
Z Sauerstoff bedeutet,
$R_1$ für Wasserstoff, Methyl oder Ethyl steht,
$R_2$ Ethyl, Cyclopropyl, oder 2-Fluorethyl bedeutet,
R3 für Wasserstoff oder Methyl steht und
$R_4$ für Wasserstoff oder Methyl steht.

Besonders bevorzugt werden Verbindungen der Formel I oder II verwendet in denen

X für N, C-H oder C-F steht,
Z Sauerstoff bedeutet,
$R_1$ Wasserstoff, Methyl oder Ethyl,
$R_2$ Cyclopropyl,
$R_3$ Methyl,
$R_4$ Wasserstoff und/oder Methyl bedeuten.

Insbesondere seien genannt:
1-Cyclopropyl-6-fluor-1,4-dihydro-4-oxo-7-(1-piperazinyl-3-chinolincarbonsäure,
1-Cyclopropyl-7-(4-ethyl-1-piperazinyl)-6-fluor-1,y-dihydroxy-y-oxo-3-chinolincarbonsäure 1-Ethyl-6-fluor-1,4-dihydro-4-oxo-7-(1-piperazinyl)-3-chinolincarbonsäure,
1-Ethyl-6-fluor-1,4-dihydro-4-oxo-7-(1-piperazinyl)-1,8-naphthyridin-3-carbonsäure,
9-Fluor-3-methyl-10-(4-methyl-1-piperazinyl)-7-oxo-2,3-dihydro-7,4-pyrido[1,2,3-de]1,4-benzoxazin-6-carbonsäure.

Wie bereits erwähnt liegen die Verbindungen der Formel I oder II in den erfindungsgemäßen Zubereitungen in Form ihrer Salze mit Basen vor. Zu den Basen zählen anorganische und organische Basen die in den Anwendungskonzentrationen physiologisch verträgliche Salze bilden. Es seien genannt die anorganischen Basen NaOH, KOH, $Ca(OH)_2$, Ammoniak. Es seien genannt die organischen Basen Amine wie Mono-, Di-, Trialkylamine, substituierte Amine wie Ethanolamin, cyclische Amine wie Morpholin, Piperazin, basische Aminosäuren wie Arginin, Lysin, Cholin, N-Methylglucamin.

Bevorzugt sind die folgenden Basen:
NaOH, KOH, Ethanolamin, Lysin, N-Methylglucamin, Arginin.

Besonders bevorzugt sind die folgenden Basen:
NaOH, KOH, Arginin.

Als Lösungsmittel für die erfindungsgemäßen Zubereitungen dient Wasser. Gegebenenfalls können auch Gemische von Wasser mit anderen Lösungsmitteln zum Einsatz kommen. Zu den Lösungsmitteln zählen: Alkohole wie ein- oder mehrwertige primäre, sekundäre oder tertiäre Alkanole wie z.B. Ethanol, Butanol, Benzylalkohol, Glykol, Glycerin, Propylenglykol, sowie N-Methylpyrrolidon.

Bevorzugt sind die folgenden Alkohole:
Ethanol, Butanol, Glycerin.

Besonders bevorzugt sind die folgenden Alkohole:
Butanol, Benzylalkohol.

Die Konzentration der Lösungsmittel in den erfindungsgemäßen Zubereitungen liegt bei 1-30 Gew.%, bevorzugt zwischen 1-10 Gew.% ganz besonders bevorzugt zwischen 1-3 Gew.%.

Die erfindungsgemäßen Zubereitungen können mit Üblichen Hilfsstoffen versetzt sein. Als solche kommen nicht-toxische pharmazeutische Stoffe wie Verdünnungsmittel, Resorptionsbeschleuniger, Resorptionshemmer, Kristallisationsverzögerer, Komplexiermittel, Antioxidantien, Konservierungsmittel und Protonisierungsmittel in Frage.

Besonders bevorzugt seien genannt:
Konservierungsmittel z.B. p-Hydroxy-benzoesäurester oder Phenole, Antioxidantien z.B. Natrium-meta-bisulfit oder Natriumsulfit, Komplexiermittel wie Natriumsalze der Aethylendiamintetraessigsäure und Kristallisationsverzögerer wie Polyvinylpyrrolidon.

Die Konzentration der Hilfsstoffe in den erfindungsgemäßen Zubereitungen liegt im allgemeinen 0,1 - 10 Gew.%, bevorzugt bei 1-2 Gew.%.

Die basischen Salze der Verbindungen der Formel I liegen in den erfindungsgemäßen Zubereitungen im allgemeinen in Konzentrationen von 0,1-30 Gew.%, bevorzugt je nach Art der Anwendung zwischen 0,5-10 Gew.% oder 0,2-2 Gew.% oder 10-30 Gew.%. Besonders bevorzugt sind Lösungen mit 0,5-10 Gew.%.

Neben den Salzen liegen Überäquimolare Mengen an Basen in den erfindungsgemäßen Zubereitungen vor. Bevorzugt sind dabei die Basen die auch zur Bildung der Salze eingesetzt werden. Der Überschuß an Base liegt dabei zwischen 0,01 - 100 me/l, bevorzugt zwischen 0,1 - 50 me/l, besonders bevorzugt zwischen 0,5-30 me/l (me = Milliäquivalent in Gramm/liter)`

Der pH-Wert der erfindungsgemäßen Zubereitung liegt zwischen 8 - 12,5, bevorzugt zwischen 9 - 11.

Zur Herstellung der erfindungsgemäßen Zubereitungen kann man von den Salzen der Verbindungen der Formel I oder II oder deren Hydraten ausgehen. Die Salze werden in der gewünschten Menge in Wasser gelöst und gegebenenfalls mit weiterer Base versetzt.

Man kann aber auch die Salze direkt in der Lösung herstellen, und zwar durch Zugabe der zur Salzbildung erforderlichen Mengen Base zu den Verbindungen der Formel I oder II.

Bevorzugt wurden die wäßrigen basischen Zubereitungen der Carbonsäuren der Formel I oder II derart hergestellt, daß man

a) den Wirkstoff gegebenenfalls zusammen mit einem Formulierungshilfsmittel in Wasser eingibt, eine anorganische oder organische Base langsam dazu rührt, bis sich ein pH-Wert von 8 bis 12,5 eingestellt hat und nach Auflösen des Wirkstoffs gegebenenfalls eine weitere Menge Wasser zugibt, bis die gewünschte Konzentration erreicht ist,

oder

b) den Wirkstoff und gegebenenfalls Formulierungshilfsmittel in Wasser suspendiert, unter Rühren eine anorganische oder organische Base hinzufügt, bis der Wirkstoff in Lösung gegangen ist und solange weiterrührt, bis ein klares, klumpenfreies Gel entstanden ist und anschließend gegebenenfalls auf die gewünschte Konzentration mit Wasser verdünnt,

oder

c) die Verbindungen der Formel I oder II in Form ihrer Salze mit Basen in Wasser auflöst und gegebenenfalls mit einem Überschuß einer Base den pH-Wert auf 8 bis 12,5 einstellt.

In dieser Weise kann man sowohl gebrauchsfertige Lösungen der aktiven Substanz, abgefüllt in geeignete Behälter, zum Beispiel in Ampullen, Injektions- oder Infusionsflaschen.

Die erfindungsgemäßen Lösungen sollen ebenso wie die ihnen zugrunde liegenden Verbindungen der Formel I oder II als Arzneimittel zur Bekämpfung bakterieller Infektionen im Human- oder Veterinärbereich verwendet werden. Als Anwendung kommen Injektionen und Infusionen in Frage. Die Dosierungen entsprechen denjenigen, die für die bekannten Verbindungen der Formel I bekannt sind.

## Beispiele

Allgemeine Vorschrift I:

In den folgenden Beispielen werden als Wirkstoffe 1-Cyclopropyl-6-fluor-1,4-dihydro-4-oxo-7-(4-ethyl-1-piperazinyl-)chinolin-3-carbonsäure (I) und 1-Cyclopropyl-6-fluor-1,4-dihydro-4-oxo-7-(1-piperazinyl) chinolin-3-carbonsäure (II) eingesetzt.

Der Wirkstoff wird zu einem Teil der angegebenen Menge Wasser und den Hilfsstoffen gegeben, die Base langsam dazugerührt bis sich der angegebene pH-Wert eingestellt hat (kontrolliert mit Glaselektrode). Während der Zugabe der Base löst sich der Wirkstoff. Nach Auflösen des Wirkstoffes wird der Rest des Wassers zugegeben und die Lösung steril filtriert.

## Beispiel 1

| | |
|---|---|
| Wirkstoff (I) | 0,5 g |
| Benzylalkohol | 1,5 g |
| Kaliumhydroxid bis pH 11 | 0,082 g |
| Wasser für Injektionszwecke | ad 100 ml |

## Beispiel 2

| | |
|---|---|
| Wirkstoff II | 30,00 g |
| Kaliumhydroxid bis pH 11 ca. | 4,50 g |
| Benzylalkohol | 1,5 g |
| Wasser für Inj.-zwecke | ad 100 ml |

## Beispiel 3

| | |
|---|---|
| Wirkstoff I | 10,00 g |
| Natronlauge 1N bis pH 11 ca | 29,00 g |
| n-Butanol | 3,00 g |
| Wasser für Inj.-zwecke ad | 100 ml |

## Beispiel 4

| | |
|---|---|
| Wirkstoff II | 5,00 g |
| n-Butanol | 3,00 g |
| Polyvinylpyrrolidon 25 | 10,00 g |
| Kaliumhydroxid bis pH 11 ca | 0,79 g |
| Wasser für Inj.-zwecke ad | 100 ml |

## Beispiel 5

| | |
|---|---|
| Wirkstoff I | 5,00 g |
| N-Methylglukamin | 6,50 g |
| Wasser für Inj.-zwecke ad | 100 ml |

pH der Lösung: 10,5.

## Beispiel 6

| | |
|---|---|
| Wirkstoff I | 20,00 g |
| Benzylakohol | 1,00 g |
| Kalilauge 10 % | 30,56 g |
| Hydroxypropylmethylcellulose-phthalat | 2,00 g |
| entmin. Wasser | 53,94 g |
| 100 ml ≙ | 107,50 g |

## Ansprüche

1. Infusions- und Injektionslösungen von Carbonsäuren der allgemeinen Formel I oder II

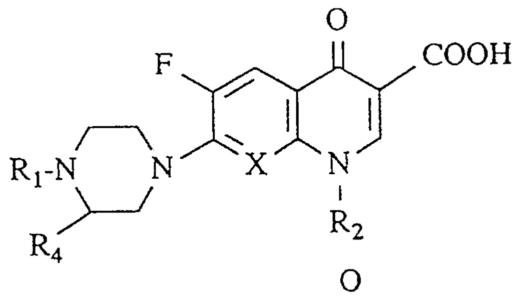

(I)

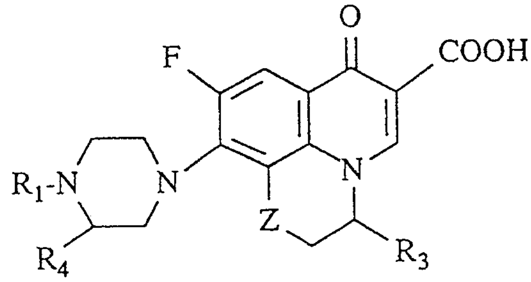

(II)

in denen

| | |
|---|---|
| X | N, C-H, C-F, |
| Z | O oder $CH_2$ |
| $R_1$ | Wasserstoff, Methyl, Ethyl oder $\beta$-Hydroxyethyl, |
| $R_2$ | Cyclopropyl, 2-Fluorethyl oder Ethyl |
| $R_3$ | Wasserstoff oder Methyl und |
| $R_4$ | H oder Methyl bedeutet, |

dadurch gekennzeichnet, daß sie die Verbindungen der Formel I oder II in Form ihrer Salze mit Basen in Gegenwart eines Überschusses an Base von 0,01 bis 100 me/l, sowie gegebenenfalls in Gegenwart weiterer Hilfsstoffe enthalten und der pH der Zubereitungen zwischen 8 und 12,5 liegt.

**2.** Zubereitungen gemäß Anspruch 1, die Salze von 1-Cyclopropyl-6-fluor-1,4-dihydro-4-oxo-7-(1-piperazinyl)-3-chinolincarbonsäure oder 1-Cyclopropyl-7-(4-ethyl-1-piperazinyl)-6-fluor-1,4-dihydro-4-oxo-3-chinolincarbonsäure enthalten.

**3.** Zubereitungen gemäß Anspruch 1, die die basischen Salze der Verbindungen der Formel I oder II in Konzentrationen von 0,1 - 30 Gew.% enthalten.

**4.** Zubereitungen gemäß Anspruch 1, die die basischen Salze der Verbindungen der Formel I oder II in Konzentrationen von 0,5 - 10 Gew.% enthalten.

**5.** Verfahren zur Herstellung von Infusions- und Injektionslösungen von Carbonsäuren der allgemeinen Formel I oder II

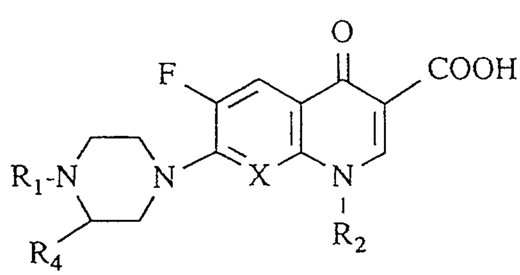

(I)

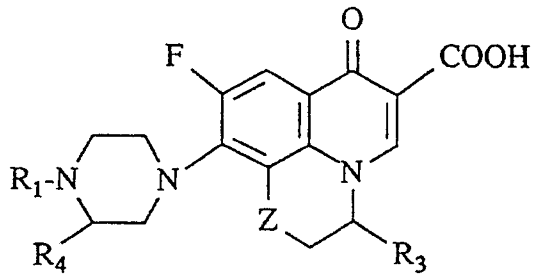

(II)

in denen

X N, C-H, C-F,

Z O oder $CH_2$,

$R_1$ Wasserstoff, Methyl, Ethyl oder $\beta$-Hydroxyethyl,

$R_2$ Cyclopropyl, 2-Fluorethyl oder Ethyl,

$R_3$ Wasserstoff oder Methyl und

$R_4$ H oder Methyl bedeutet,

dadurch gekennzeichnet, daß man den Wirkstoff gegebenenfalls zusammen mit einem Formulierungshilfsmittel in Wasser eingibt, eine anorganische oder organische Base im Überschuß von 0,01 bis 100 me/l langsam dazurührt, bis sich ein pH-Wert von 8 bis 12,5 eingestellt hat und nach Auflösen des Wirkstoffs gegebenenfalls eine weitere Menge Wasser zugibt, bis die gewünschte Konzentration erreicht ist.

## Claims

1. Infusion and injection solutions of carboxylic acids of the general formula I or II

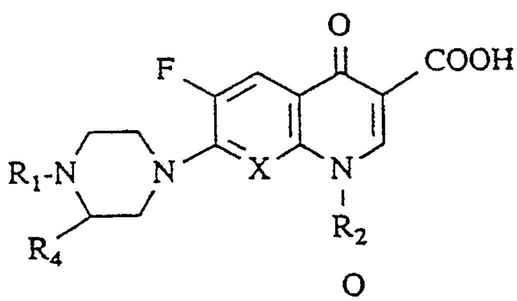

(I)

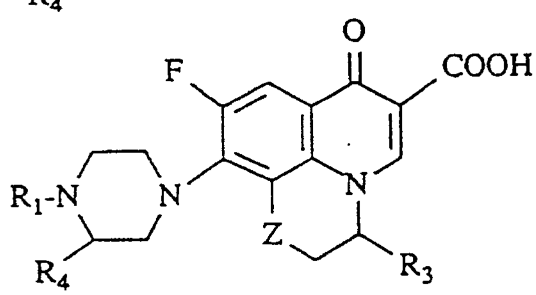

(II)

in which

X denotes N, C-H or C-F,

Z denotes O or $CH_2$,

$R_1$ denotes hydrogen, methyl, ethyl or $\beta$-hydroxyethyl,

$R_2$ denotes cyclopropyl, 2-fluoroethyl or ethyl,

$R_3$ denotes hydrogen or methyl and

$R_4$ denotes H or methyl,

characterized in that they contain the compounds of the formula I or II in the form of their salts with bases, in the presence of a 0.01 to 100 ml/l excess of base, and if appropriate in

the presence of further auxiliaries and the pH of the formulations is between 8 and 12.5.

2. Formulations according to Claim 1 which contain salts of 1-cyclopropyl-6-fluoro-1,4-dihydro-4-oxo-7-(1-piperazinyl)-3-quinolinecarboxylic acid or 1-cyclopropyl-7-(4-ethyl-1-piperazinyl)-6-fluoro-1,4-dihydro-4-oxo-3-quinolinecarboxylic acid.

3. Formulations according to Claim 1 which contain basic salts of the compounds of the formula I or II in concentrations of 0.1 - 30% by weight.

4. Formulations according to Claim 1 which contain the basic salts of the compounds of the formula I or II in concentrations of 0.5 - 10% by weight.

5. Process for the preparation of infusion and injection solutions of carboxylic acids of the general formula I or II

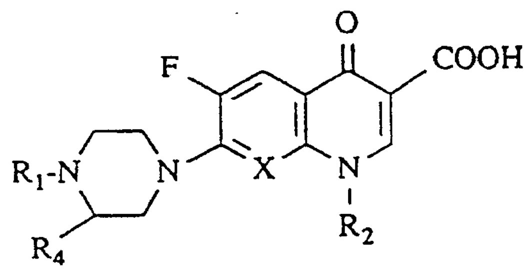

(I)

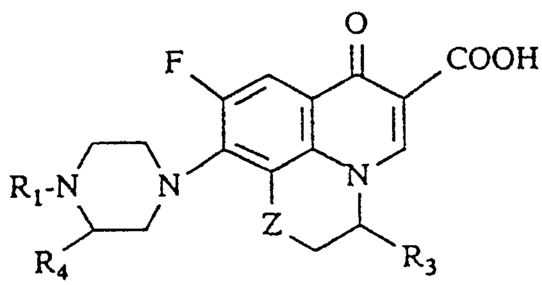

(II)

in which

X denotes N, C-H or C-F,

Z denotes O or $CH_2$,

$R_1$ denotes hydrogen, methyl, ethyl or $\beta$-hydroxyethyl

$R_2$ denotes cyclopropyl, 2-fluoroethyl or ethyl,

$R_3$ denotes hydrogen or methyl and

$R_4$ denotes H or methyl,

characterized in that the active compound is introduced into water, if appropriate together with a formulation auxiliary, an inorganic or organic base in an excess of 0.01 to 100 ml/l is slowly stirred in until a pH value of 8 to 12.5 has been established and, after the active compound has been dissolved, a further amount of water is added, if appropriate, until the desired concentration is reached.

**Revendications**

1. Solutions perfusables et injectables d'acides carboxyliques de formule générale I ou II

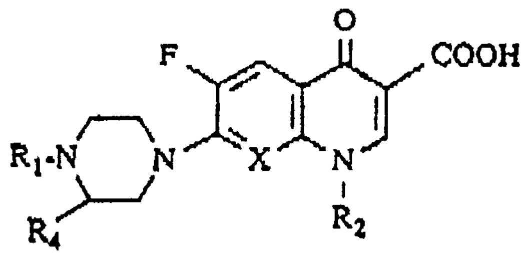

(I)

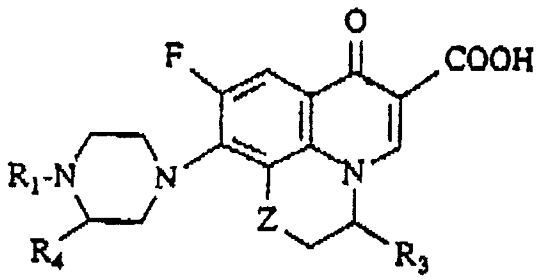

(II)

dans lesquelles

- X représente N, C-H, C-F,
- Z représente O ou $CH_2$,
- $R_1$ représente l'hydrogène, un groupe méthyle, éthyle ou $\beta$-hydroxyéthyle,
- $R_2$ représente un groupe cyclopropyle, 2-fluoréthyle ou éthyle,
- $R_3$ représente l'hydrogène ou un groupe méthyle et
- $R_4$ représente H ou un groupe méthyle,

caractérisés en ce qu'ils contiennent les composés de formule I ou II sous forme de leurs sels avec des bases, en présence d'un excès de base de 0,01 à 100 me/l, ainsi qu'éventuellement en présence d'autres adjuvants, et que le pH des préparations se situe entre 8 et 12,5.

**2.** Préparations selon la revendication 1, contenant des sels d'acide 1-cyclopropyl-6-fluoro-1,4-dihydroxy-4-oxo-7(1-pipérazinyl)-3-quinoléinecarboxylique ou d'acide 1-cyclopropyl-7-(4-éthyl-1-pipérazinyl)-6-Fluoro-1,4-dihydro-4-oxo-3-quinoléinecarboxylique.

**3.** Préparations selon la revendication 1, contenant des sels basiques des composés de formule I ou II dans des concentrations de 0,1 à 30% en poids.

**4.** Préparations selon la revendication 1, contenant des sels basiques des composés de formule I ou II dans des concentrations de 0,5 à 10% en poids.

**5.** Procédé pour la fabrication de solutions perfusables et injectables d'acides carboxyliques de formule générale I ou II

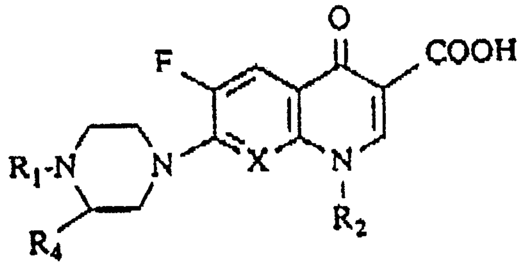

(I)

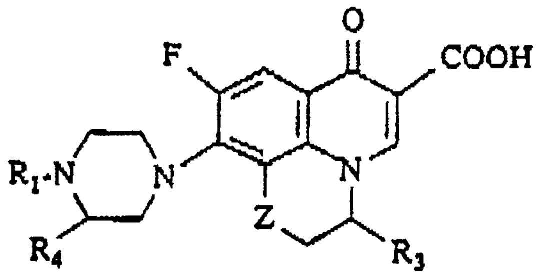

(II)

dans lesquelles

X représente N, C-H, C-F,

Z représente O ou $CH_2$,

$R_1$ représente l'hydrogène, un groupe méthyle, éthyle Ou $\beta$-hydroxyéthyle,

$R_2$ représente un groupe cyclopropyle, 2-fluoréthyle ou éthyle,

$R_3$ représente l'hydrogène ou un groupe méthyle et

$R_4$ représente H ou un groupe méthyle,

caractérisés en ce qu'on introduit la substance active dans l'eau éventuellement avec un adjuvant de formulation, qu'on y ajouta lentement en agitant une base inorganique ou organique en excès de 0,01 à 100 me/l, jusqu'à ce qu'un pH de 8 à 12,5 soit établi, et on ajoute éventuellement encore de l'eau après la dissolution de la substance active jusqu'à ce que la concentration désirée soit atteinte.